# EUROPEAN PATENT APPLICATION

(11) **EP 3 854 349 A1**
(43) Date of publication of application: **28.07.2021**
(21) Application number: 20315007.3
(22) Date of filing: 23.01.2020
(51) Int. Cl.: A61F 2/07

(54) **ENDOPROSTHESIS AND METHOD OF MANUFACTURING AN ENDOPROSTHESIS**

(71) Applicant: Kardiozis SAS, 13100 Aix-en-Provence (FR)
(72) Inventor: VANDAELE FENOUIL, Nathalie, 13100 Aix-en-Provence (FR); BURG, Brian, 13100 Aix-en-Provence (FR); CHAPUT, Oriane, 13100 Aix-en-Provence (FR)
(74) Representative: Hepp Wenger Ryffel AG

(57) **Abstract**

The invention relates to an endoprosthesis (60), in particular a vascular stent or a heart stent. The endoprosthesis comprises a stent structure (2) with a stent surface (3) and has at least one fiber (1) arranged on the stent surface (3). The stent structure (2), preferably the stent surface (3), comprises an attachment mechanism (4,5,6,7) to directly attach a fiber (1) on the stent.

## Description

The present invention relates to an endoprosthesis and a method of manufacturing an endoprosthesis according to the preamble of the independent claims.

Endoprostheses, in particular vascular and heart stents, are used to support blood vessels in the human body. For example, occlusions or aneurysms can be treated by placing such an endoprosthesis at the respective treatment site. In the treatment of an occlusion, the endoprosthesis keeps the vessel open for unhindered blood flow. In the case of an aneurysm, the endoprosthesis can prevent circulation of blood into the aneurysm and thus lower the risk of a thrombus, rupture or further growth of the aneurysm.

It is known in the prior art to use thrombogenic elements on endoprostheses. For example, WO 2013/182614 A1 discloses an endoprosthesis with thrombogenic elements that extend away from a body of the endoprosthesis and promote thrombosis. This allows for the occlusion of an aneurysm to enhance the above-mentioned treatment effect.

WO 2006/0166167 discloses fibers arranged on an endovascular prosthesis.

WO 2019/122944 discloses the attachment of a strip of fabric on with fibers on a stent graft.

DE 195 31 659 discloses a stent wire which comprises fibers. The fibers are attached to the wire structure by being held between two wires that form a spiral. In particular, the fibers of DE 195 31 659 are arranged on the wire used to produce the stent structure. They cannot be attached to the stent post-production.

Currently known methods hence do not provide a simple way of post-production arrangement of thrombogenic elements on an endoprosthesis, in particular on a stent structure. Fixation and attachment of thrombogenic elements is usually cumbersome and difficult, and not typically versatile. In addition, prior art solutions are limited to generic thrombus generation means that are not adapted to patient-specific needs.

Thus, the object of the present invention is to overcome the drawbacks of the prior art, in particular to provide a mechanism to attach a fiber to an endoprosthesis in a simple, cheap and reliable way.

This and other objects are achieved by the endoprosthesis and the methods according to the characterizing portion of the independent claims.

The endoprosthesis according to the invention is preferably a vascular stent or a heart stent. It comprises a stent structure with a stent surface. The endoprosthesis further has at least one fiber arranged on the stent surface. The stent structure, in particular the stent surface, comprises an attachment mechanism to directly attach a fiber on the stent. The attachment mechanism may in particular be adapted for attachment of a fiber after production of the stent structure. In particular, the attachment may be reversible. This allows for a post-production arrangement of a fiber on a stent structure. For example, different stents may be produced without fibers and fibers may then be attached depending on a particular patient's need. It is also possible to attach different types of fibers on the stent structure, in particular on the stent surface. In addition, it is particularly advantageous to arrange the fiber on the stent structure as opposed to a graft structure because a stent provides a higher mechanical stability and is easier to image. If fibers are arranged on a stent structure, it is therefore easier, simpler, cheaper, and safer to place fiber at a desired position in the body.

The attachment mechanism may comprise at least one of a mechanical attachment, a physical attachment, and a chemical attachment. A mechanical attachment may encompass a structure on the stent surface that mechanically engages the fiber, for example a hook. It may be a form fit or a force fit. A physical attachment may encompass any form of physical bonding, such as van-der-Waals interaction, electrostatic forces, and magnetic forces. A chemical attachment in particular encompasses chemical crosslinking and an attachment mechanism involving a chemical reaction.

Alternatively or additionally, the attachment mechanism may comprise at least one groove in the stent structure. A groove provides a pre-determined position for a fiber to be arranged. It also provides a recess such that the attached part of the fiber is less exposed because it is depressed in the stent structure. The groove therefore provides a safer attachment. It is possible to further functionalize the groove surface with any of the attachment mechanisms disclosed herein.

Alternatively or additionally, the attachment mechanism may also comprise at least one slot in the stent structure. A slot comprises an opening in the stent structure, whereas a groove can also be a recess in the stent material. A slot provides even safer attachment for a fiber. In particular, a slot provides a very easy way to attach a fiber e.g. via a knot.

The stent surface may also comprise a surface structure which is part of the attachment mechanism. The surface structure provides frictional attachment to the fiber. Frictional attachment may encompass a particular material selection to increase friction between the fiber and the stent surface, or a particular surface treatment to increase the friction between the fiber and the stent surface. Enhanced friction between the fiber and the stent structure may be sufficient to attach the fiber to the stent structure. However, it is possible to combine frictional attachment with other mechanisms, or to only use frictional attachment as an additional support mechanism, for example in a groove, a slot, or with knots.

The surface structure may comprise a surface with increased roughness. The surface roughness may be increased by mechanical abrasion, such as sandblasting, milling or grinding. In particular, the surface roughness may be increased on an outer portion of the stent surface as compared to an inner portion of the stent surface. The surface roughness may also be uniform over the entire stent surface. Additionally or alternatively, surface inhomogeneities may be attached to the stent surface to increase surface roughness, so that there are parts of the surface having an increased roughness as compared to neighbouring parts not intended for fixation of fibers. In particular, the surface roughness (measured as average peak-to-valley height, or Rₐ, as known by the person skilled in the art) of parts intended for the attachment of fibers may be 50-200% higher compared to the surface roughness of parts not intended for attachment of fibers. In particular, the surface roughness may be increased as compared to a surface of electropolished Nitinol.

The surface structure may comprise a directional abrasion. Directional abrasion in particular combines the advantages of groves and increased surface roughness. Directional abrasion provides higher friction between the stent structure or stent surface and the fiber. The groove structure may accept a fiber. In addition, a directional abrasion may be used if a preferred orientation of the fibers on the stent surface is desirable. For example, a directional abrasion may be achieved by brushing the stent surface. As such, a directional abrasion shall in particular encompass scratches on the stent surfaces with a preferred orientation, wherein the orientation of the scratches is not necessarily identical.

The stent structure, in particular the stent surface, is coated with an attachment material. The attachment material may preferably be miscible with the fiber material, in particular in their respective molten states. For example, the attachment material may be a first polymer blend, and the fiber may consist of a second polymer blend, wherein both the first and the second polymer blends are miscible. Therefore, if the fiber and the attachment material are heated up such that at least one of them, preferably both, are at least partially melted, the fiber material and the attachment material will mix and be fixedly attached to one another upon cooling.

The fiber further may also comprise an anchor, particularly an anchor that is arranged at a free end of the fiber. The anchor may be a thicker portion of the fiber made of the same material or a different material, in particular a different material with adapted mechanical properties. For example, the anchor may be stiffer or more elastic than the fiber. A thicker portion of the fiber may in particular provide mechanical retention in case of a form fit, e.g. if the fiber is arranged within a hole, a slot, or a similar recess in the stent structure. Additionally or alternatively, the anchor may also be a structure that provides an attraction force to the stent structure, for example a magnetic force, an electrostatic force, or an adhesive force.

The stent structure may comprise an adaptable material that is adaptable such as to attach, in particular permanently, to the fiber by application of at least one treatment of a group comprising of plasma treatment, heat treatment, a radiation treatment and pressure treatment. In particular, the stent structure and /or the stent surface may undergo a phase transformation upon such a treatment. Similarly, the treatment may activate an adhesive layer, induce diffusion between the fiber and the stent structure, or induce melting.

The invention further relates to a method of attaching a fiber to an endoprosthesis. Preferably, the method is performed to attach a fiber to an endoprosthesis as disclosed herein. The method comprises a step of providing a stent structure having a stent surface. A fiber is attached to at least one of the stent structures and the stent surface. The attachment of the fiber is performed by at least one of welding, gluing, heating by exposure to electromagnetic radiation, and mechanical attachment. The method provides a particularly advantageous way of attaching a fiber to a stent structure after production of the stent. While the method is particularly suited to attach a fiber to a stent structure as described herein, the person skilled in the art understands that the method may be used to attach a fiber to any stent known in the art. Of course, the person skilled in the art also understands that it may be advantageous to provide the stents with any combination of the features described herein.

In the following, the invention is described in detail with reference to the following figures, showing:
- Fig. 1a-1c:: a first embodiment of a stent structure with a groove for fiber attachment in a cross-sectional view and a side view.
- Fig. 2a-2b:: a second embodiment of a stent structure with groove for fiber attachment in a cross-sectional view and a side view.
- Fig. 3a-3c:: a third embodiment of a stent structure with a slot for fiber attachment in a cross-sectional view and a side view.
- Fig. 4a-4c:: a fourth embodiment of a stent structure with a surface structure for fiber attachment in a cross-sectional view and a side view.
- Fig. 5a-5c:: a fifth embodiment of a stent structure with an attachment material for fiber attachment in a cross-sectional view and a side view.
- Fig. 6a-6c:: a sixth embodiment of a stent structure with a hole for fiber attachment in a cross-sectional view and a side view.
- Fig. 7a-7c:: the sixth embodiment of a stent structure in a cross-sectional view and a side view with a fiber comprising an anchor element.
- Fig. 8a-8c:: a seventh embodiment of a stent structure adapted for fiber attachment through pressure in a cross-sectional view and a side view.
- Fig. 9a-9c:: an eighth embodiment of a stent structure adapted for fiber attachment through plasma treatment in a cross-sectional view and a side view.
- Fig. 10a-10c:: a ninth embodiment of a stent structure adapted for fiber attachment comprising a directional abrasion in a cross-sectional view and a side view.
- Fig. 11:: a tenth embodiment of a stent structure adapted for fiber attachment through elevated temperatures in a cross-sectional view and a side view.
- Fig. 12:: an eleventh embodiment of a stent structure adapted for fiber attachment without post-manufacturing modification in a cross-sectional view and a side view.
- Fig. 13:: an endoprosthesis comprising fibers attached on a stent structure.

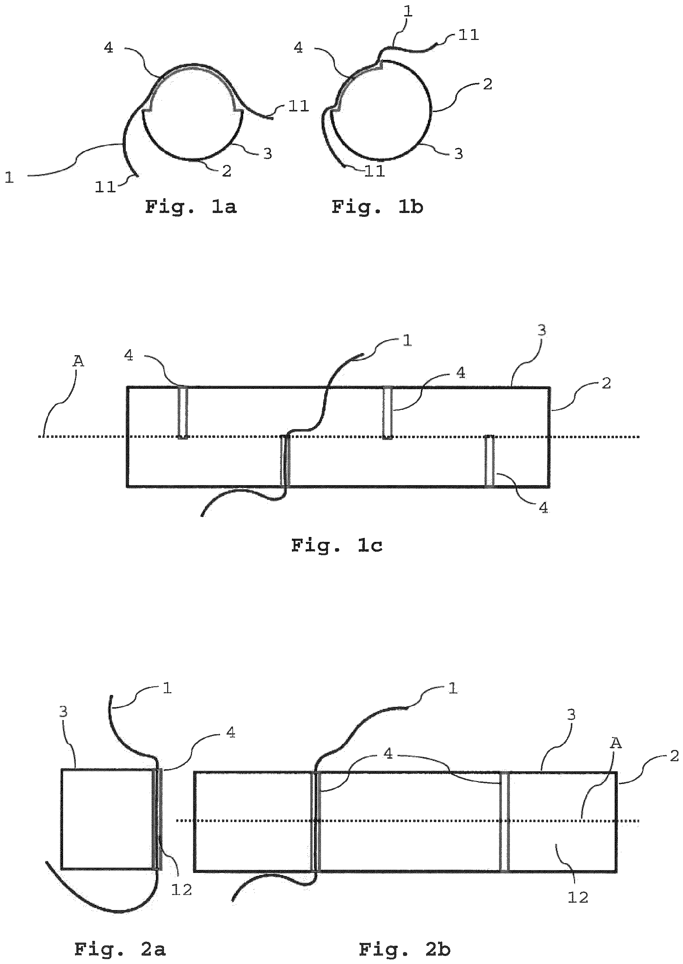

Fig. 1a shows a stent structure 2 in a cross-sectional view in a plane perpendicular with respect to a longitudinal axis A (see Fig. 1c). The stent structure 2 is typically a strut or a bar of a stent. It is generally known to the skilled person to build an expandable stent out of a tube by laser cutting such as to form a scaffold. The stent structure 2 shown in figure 1a and the various stent structures shown in the subsequent figures refer to one single strut or bar of a stent. It will be understood by the skilled person, that similar structures may be provided in other or also in all of the struts or bars of a stent. The stents shown herein below can be self-expandable or balloon expandable. They can be covered by a graft material in a manner known to the skilled person. Typically, the graft material may be provided with holes, so that the fibers attached to the stent (as will be shown herein below) can be extending through the holes in the graft material.

A recessed groove 4 is arranged on the stent surface 3 and oriented substantially perpendicularly to the longitudinal axis of the stent structure 2. The groove 4 has a length corresponding to half a circumference of the stent structure 2. A fiber 1 is partially arranged within the groove 4. The fiber has two free ends 11 that extend away from the stent structure, while a middle portion of the fiber 1 is arranged within the groove. The stent structure 2 comprises longitudinally extending bar or strut like elements and is made in a manner known to the skilled person and is e.g. made of a Nitinol alloy with a particularly adapted surface 4 that provides high friction to the fiber made of Dacron. Therefore, the fiber is retained in the groove.

Fig. 1b shows a similar stent structure 2 as Fig. 1a. The groove 4 in the shown embodiment, however, only has a length corresponding to about a quarter of a circumferential length of the stent structure 2. In addition, the embodiment of Fig. 1b does not have a particularly adapted surface. The fiber 1 is therefore retained only by friction and electrostatic attraction.

Fig. 1c shows the stent structure 2 of Fig. 1a in a side view. The stent structure comprises four grooves 4 arranged perpendicularly with respect to the longitudinal axis. The grooves 4 are arranged at a distance between each other. Two neighbouring grooves 4 are oriented on opposite sides of the stent structure 1, i.e. one groove is arranged at an angle of 180° compared to the other groove. In the shown embodiment, one of the four grooves 4 has a fiber attached. The other three grooves 4 may be used to attach other fibers 1 if necessary and desired for any given application. Alternatively, the stent structure 2 may be used to treat a patient with only one fiber 1.

Fig. 2a shows a similar stent structure 2 as Fig. 1b. The stent structure has rectangular cross-section. The stent structure 2 comprises a groove 4 on a side wall 12 of the stent structure.

Fig. 2b shows the stent structure of Fig. 2a in a side view. The stent structure 2 in this embodiment comprises two grooves 4 on the side wall 12. The grooves are oriented perpendicularly with respect to the longitudinal axis A of the stent structure. It would be possible to arrange more grooves 4 on different side walls. However, the shown embodiment only comprises grooves 4 on the side wall 12 shown here.

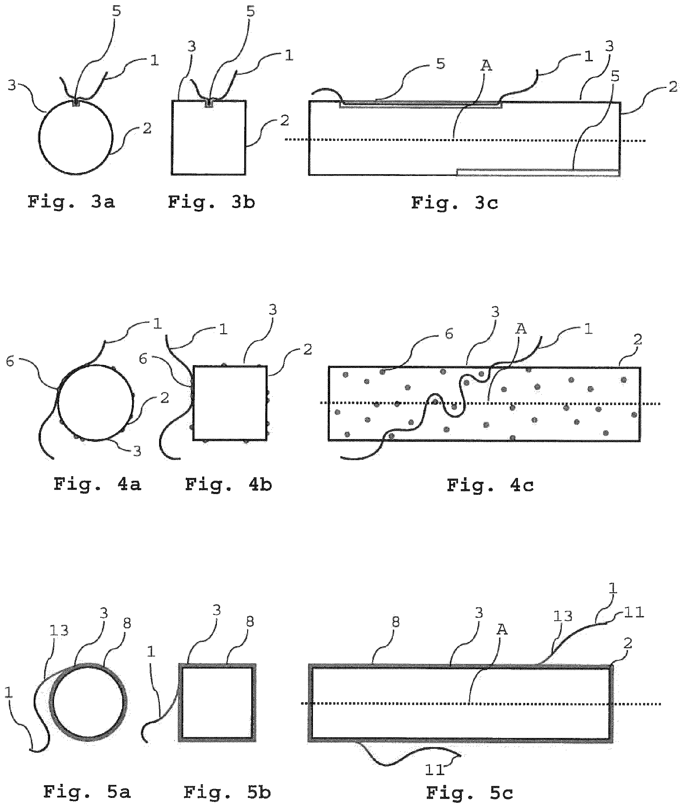

Fig. 3a shows an embodiment of a stent structure in a cross-sectional view in a plane perpendicular to the longitudinal axis A of the stent structure 2. The shown embodiment has a circular circumference. The stent structure 2 comprises a slot 5 on the stent surface 3 that is adapted to attach a fiber 1. Here, the fiber 1 is pushed into the slot 5. Because the stent structure 2 is made of a Nitinol alloy, it is thus elastic and the metal structure of the stent structure 2 presses against the fiber 1 and holds it in place.

Fig 3b shows a similar stent structure 2 as shown in Fig. 3a. The stent structure 2 has a rectangular cross-section, however.

Fig. 3c shows a similar stent structure 2 as in Fig. 3a in a side view. The stent structure 2 comprises two slots 5. Here, the fiber is arranged substantially in parallel with respect to the longitudinal axis A of the stent structure 2. The attachment mechanism in the slot 5 is similar to the one of Fig. 3a, except that the fiber 1 penetrates through the slot entirely at a first penetration position to the inside of the stent structure 2 and penetrates back out at a second penetration position arranged at a distance to the first penetration position. A second slot 5 is arranged on stent structure 2. Both slots 5 are arranged in parallel to the longitudinal axis A of the stent structure 2. Of course, it is also possible to arrange the fibres at an angle with respect to the axis of the stent structure. Typically, the angle may be between 0° and 90° with respect to the axis A of the stent structure.

Fig. 4a shows an embodiment of a stent structure in a cross-sectional view in a plane perpendicular to the longitudinal axis A of the stent structure 2. The shown embodiment has a circular circumference. The stent surface 3 comprises an abrasive material 6 made of biocompatible iron oxide coated onto the surface 3 of the stent structure 2. As consequence, the stent surface 2 has an increased roughness and a higher specific surface area. The higher specific surface area also provides for electrostatic attraction and van-der-Waals interaction which provide further attachment.

Fig 4b shows a similar stent structure 2 as shown in Fig. 4a. The stent structure 2 has a rectangular cross-section, however.

Fig. 4c shows the stent structure 2 of Fig. 4a in a side view. The fiber 1 is attached to the surface by means of the abrasive 6 coated on the stent surface 3. The fiber 1 thus has no particular orientation on the stent surface and is rather attached in a random manner depending on the position of the abrasive particles 6.

Fig. 5a shows an embodiment of a stent structure in a cross-sectional view in a plane perpendicular to the longitudinal axis A of the stent structure 2. The shown embodiment has a circular circumference. The stent surface 3 at least in the area of the stent structure 2 is coated with a layer of polyethylene as an attachment material 8. A fiber 1, consisting of polyethylene terephthalate, is attached to the stent structure 2 via the attachment material 8. An intermediary portion 13 is formed between the fiber 1 and the attachment material 8 due to the attachment process. The fiber 1 is attached to the attachment material 8 by heating, upon which the fiber 1 partially melts and mixes with the attachment material 8. Upon cooling the fiber 1 is fixedly attached to the attachment material 8.

Fig 5b shows a similar stent structure 2 as shown in Fig. 5a. The stent structure 2 has a rectangular cross-section, however.

Fig. 5c shows the stent structure 2 of Fig. 5a in a side view. Two fibers 1 are attached to the stent structure 2 via the attachment material. The fibers 1 only have one free end 11 each extending away from the stent structure 2 as they are attached to the attachment material 8 via the other free end.

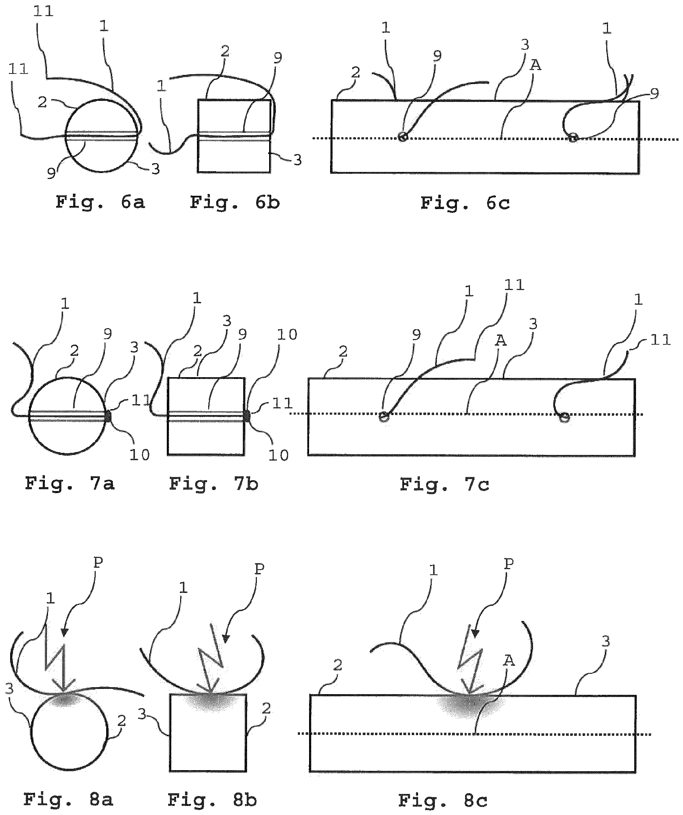

Fig. 6a shows an embodiment of a stent structure in a cross-sectional view in a plane perpendicular to the longitudinal axis A of the stent structure 2. The shown embodiment has a circular circumference. A hole 9 extends through the stent structure 2 along an axis substantially perpendicular to the longitudinal axis A of the stent structure 2. A fiber 1 extends through the hole 9 such that one free end extends away from the stent structure on each side of the hole 9. A middle portion of the fiber 1 resides within the hole 9 and is retained in the hole 9 by friction between the stent structure 2 material and the fiber 1 material. It would be conceivable to additionally secure the fiber 1 to the stent structure 2.

Fig 6b shows a similar stent structure 2 as shown in Fig. 6a. The stent structure 2 has a rectangular cross-section, however.

Fig. 6c shows the stent structure 2 of Fig. 6a in a side view. The stent structure 2 comprises two holes, each of which is provided with a fiber 1.

Fig. 7a shows an embodiment of a stent structure in a cross-sectional view in a plane perpendicular to the longitudinal axis A of the stent structure 2. The shown embodiment has a circular circumference. The stent structure 2 is identical to the stent structure of Figs. 6a and 6c and comprises holes 9 across the stent structure 2. The fiber 1, by contrast, has an anchor element 10 arranged on a free end 11. The anchor element consists of a thicker pearl of the same material as the fiber, which could for example be created by partial melting and formation of a drop at the free end 11 due to surface tension. Alternatively, a bead may be arranged at the free end, or a drop of glue, or a knot could be used for that purpose. As a consequence, the fiber extends through the hole 9. The free end 11 with the anchor element 10 is too large to pass through the hole 9 and therefore fixes the fiber in the desired position.

Fig 7b shows a similar stent structure 2 as shown in Fig. 7a. The stent structure 2 has a rectangular cross-section, however.

Fig. 7c shows the stent structure 2 of Fig. 7a in a side view. The shown stent structure 2 comprises two holes 9 with one fiber 1 arranged therein each.

Fig. 8a shows an embodiment of a stent structure in a cross-sectional view in a plane perpendicular to the longitudinal axis A of the stent structure 2. The shown embodiment has a circular circumference. A fiber 1 is attached to the stent structure 2 via a pressure force P in a middle portion of the fiber 1. The fiber may also be attached at a free end, for example as the fiber 1 shown in Figs. 5a-5c. The fiber 1 comprises a metallic material that is miscible with the stent structure 2 material. Therefore, when pressure is applied, the metallic material diffuses into the stent structure and permanently attaches the fiber 1 to the stent structure. Heating of the fiber 1 and the stent structure 2 during pressure application can be used to enhance diffusion and accelerate the attachment.

Fig 8b shows a similar stent structure 2 as shown in Fig. 8a. The stent structure 2 has a rectangular cross-section, however.

Fig. 8c shows the stent structure 2 of Fig. 8a in a side view.

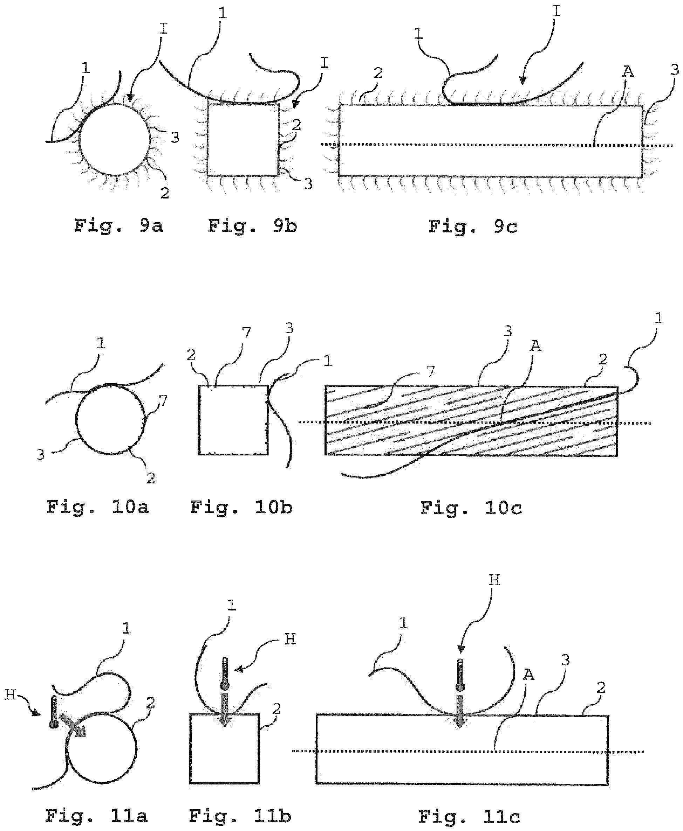

Fig. 9a shows an embodiment of a stent structure in a cross-sectional view in a plane perpendicular to the longitudinal axis A of the stent structure 2. The shown embodiment has a circular circumference. A plasma I is used to treat the stent surface 3 of the stent structure and to enhance its polarity. As a consequence, the fiber 1, which also has a highly polar surface, preferably attaches to the stent surface 3. It would also be possible to use an inverse process, where the stent surface 3 is made hydrophobic in order to attract a hydrophobic fiber 1. Such a configuration is particularly advantageous for permanent attachment of fibers 1 for use in situations where a polar fluid, such as water or blood, is present.

Fig 9b shows a similar stent structure 2 as shown in Fig. 9a. The stent structure 2 has a rectangular cross-section, however.

Fig. 9c shows the stent structure 2 of Fig. 9a in a side view.

Fig. 10a shows an embodiment of a stent structure in a cross-sectional view in a plane perpendicular to the longitudinal axis A of the stent structure 2. The shown embodiment has a circular circumference. The stent surface was brushed and thus comprises a direction abrasion. Here, the scratches 7 caused by brushing enhance electrostatic attraction due to an increase in the specific surface area of the stent structure 2.

Fig 10b shows a similar stent structure 2 as shown in Fig. 10a. The stent structure 2 has a rectangular cross-section, however.

Fig. 10c shows the stent structure 2 of Fig. 10a in a side view. The fibers 1 are preferentially attached to the stent surface 3 in the same orientation as the scratches 7 in order to maximize the interaction area between the fiber 1 and the stent surface 3.

Fig. 11a shows an embodiment of a stent structure in a cross-sectional view in a plane perpendicular to the longitudinal axis A of the stent structure 2. The shown embodiment has a circular circumference. Both the stent structure 2 and the fiber 1 comprise a weldable metal. The fiber 1 is thus attached by welding, i.e. by providing heat H to an attachment point. The fiber 1 and the stent structure 2 and/or the stent surface partially melt and form an attachment for the fiber on the stent surface 3.

Fig 11b shows a similar stent structure 2 as shown in Fig. 11a. The stent structure 2 has a rectangular cross-section, however.

Fig. 11c shows the stent structure 2 of Fig. 11a in a side view.

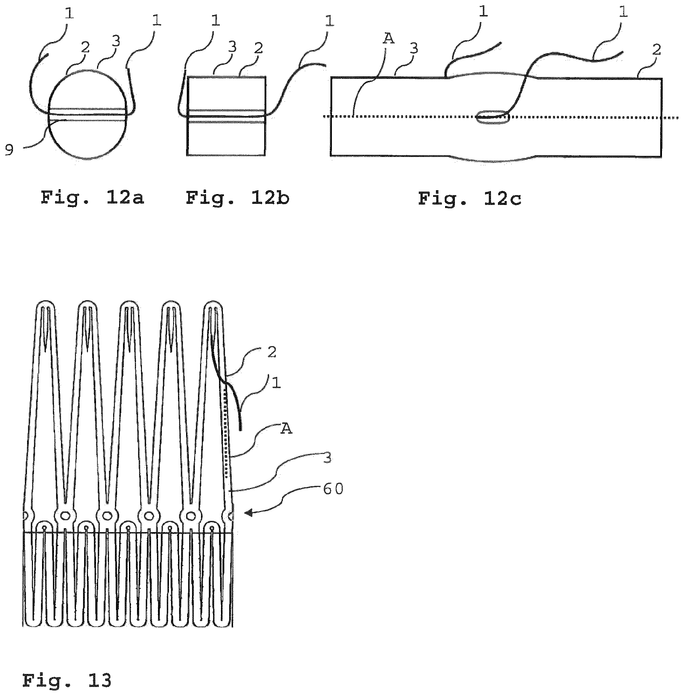

Fig. 12a shows an embodiment of a stent structure in a cross-sectional view in a plane perpendicular to the longitudinal axis A of the stent structure 2. The shown embodiment has a circular circumference. The stent structure 2 comprises a hole 9 through which the fiber extends.

Fig 12b shows a similar stent structure 2 as shown in Fig. 12a. The stent structure 2 has a rectangular cross-section, however.

Fig. 12c shows the stent structure 2 of Fig. 12a in a side view. The embodiment according to figures 12a, 12b and 12c is similar to the one of figures 6a or 6b. According to the embodiment of figure 12a, 12b or 12c, the stent structure has a shape designed to allow the passage of a fibre. The passage has an elongated cross-section (see figure 12c). However, there is no post manufacturing modification of the stent structure 2, apart from the subsequent insertion of the fibre.

Fig. 13 shows an exemplary endoprosthesis 60 according to the invention. The endoprosthesis 60 comprises the stent structure 2 in the form of a stent comprising a Z-shaped stent wire with a diameter of 0.3 mm to which fibers 1 are attached. Several fibers 1 (only one being shown) are arranged on the stent surface 3 of the stent structure 2. Any of the above-mentioned attachment mechanisms is suitable to attach the fibers 1 to the stent surface 3. In the present embodiment, all the fibers are attached by the same mechanism. However, it would also be conceivable to attach different fibers 1 with different mechanisms and thus to include several of the above-described mechanisms in one endoprosthesis. Similarly, a plurality of fibers 1 may be attached on the stent surface 2 with a substantially uniform distribution. The person skilled in the art will understand that this is merely an exemplary embodiment and that any number of fibers 1 could be attached to the surface 3, or with any distribution desired for a particular application. The fibers are preferably attached to the stent structure 2 by the method according to the invention. The stent structure 2 is typically a part of a Nitinol stent known to the skilled person. While in figure 13, a fiber 1 is only shown attached to one strut 2, of course fibers may be attached to more than one strut and also to all of the struts.

The struts 2 of the endoprosthesis 60 typically may have a diameter of 0.3 mm . The endoprosthesis 16 may be covered fully or partly by a graft material (not shown). The graft material may be provided with holes through which the fibers 1 may extend.

## Claims

1. An endoprosthesis (60), in particular a vascular stent or a heart stent, comprising a stent structure (2) with a stent surface (3), having at least one fiber (1) arranged on the stent surface (3), **characterized in that** the stent structure (2), preferably the stent surface (3), comprises an attachment mechanism (4,5,6,7) to directly, preferably reversibly, attach a fiber (1) on the stent.

2. Endoprosthesis (60) according to claim 1, wherein the attachment mechanism comprises at least one of a mechanical attachment, physical attachment, and chemical attachment.

3. Endoprosthesis (60) according to one of the claims 1 or 2, wherein the attachment mechanism comprises at least one groove (4) in the stent structure (2).

4. Endoprosthesis (60) according to one of the preceding claims, wherein the attachment mechanism (4,5,6,7) comprises at least one slot (5) in the stent structure (2).

5. Endoprosthesis (60) according to one of the preceding claims, wherein the stent surface (3) comprises a surface structure (6,7) which is part of the attachment mechanism (4,5,6,7), wherein the surface structure (6,7) provides frictional attachment to the fiber (1).

6. Endoprosthesis (60) according to claim 5, wherein the surface structure comprises a surface with an increased roughness (6).

7. Endoprosthesis (60) according to one of the claims 5 or 6, wherein the surface structure comprises a directional abrasion (7).

8. Endoprosthesis (60) according to one of the preceding claims, wherein the stent structure (2), preferably the stent surface (3), is coated with an attachment material (8), wherein the attachment material (8) is preferably miscible with the fiber material, in particular in their respective molten states.

9. Endoprosthesis (60) according to one of the preceding claims, wherein the attachment mechanism comprises a passageway (9) through the stent structure (2), preferably a passageway (9) angled with respect to the longitudinal axis (A) of the stent structure (2), even more preferably angled at an angle of 90°.

10. Endoprosthesis (60) according to claim 9, wherein the fiber further comprises an anchor (10), preferably arranged at a free end (11) of the fiber (1).

11. Endoprosthesis (60) according to any one of the preceding claims, wherein the stent structure (2) comprises an adaptable material that is adaptable such as to attach, preferably permanently, to the fiber (1) by application of at least one of plasma treatment, heat treatment (H), radiation treatment and pressure treatment (P).

12. A method of attaching a fiber (1) to an endoprosthesis (60), in particular an endoprosthesis according to one of the preceding claims, wherein a stent structure (2) comprising a stent surface (3) is provided, and a fiber (1) is attached to at least one of the stent structure (2) and the stent surface (3), **characterized in that** the attachment of the fiber (1) is performed by at least one of welding, gluing, heating by exposure to ultrasound waves, heating by exposure to electromagnetic radiation, and mechanical attachment.
